# EUROPEAN PATENT SPECIFICATION

(11) **EP 2 654 576 B1**
(45) Date of publication and mention of the grant of the patent: **12.08.2020**
(21) Application number: 11806122.5
(22) Date of filing: 23.12.2011
(51) Int. Cl.: A61B 17/04

(54) **ADJUSTABLE ANCHOR SYSTEMS**
EINSTELLBARE VERANKERUNGSSYSTEME
SYSTÈMES D'ANCRAGE AJUSTABLE

(30) Priority: 23.12.2010 US 977146
(43) Date of publication of application: 30.10.2013
(73) Proprietor: DePuy Mitek, LLC, Raynham, MA 02767 (US)
(72) Inventor: HERNANDEZ, Joseph, Raynham, MA 02767 (US); SENGUN, Mehmet, Ziya, Raynham, MA 02767 (US); MILLER, Gerome, Raynham, MA 02767 (US); WHITTAKER, Gregory, R., Raynham, MA 02767 (US); MCALISTER, Gary, B., Raynham, MA 02767 (US)
(74) Representative: Tunstall, Christopher Stephen
(86) International application number: PCT/US2011/067119
(87) International publication number: WO 2012/088496

(56) References cited:
- EP-A1- 1 199 035
- EP-A1- 1 568 326
- US-A- 4 141 087
- US-A1- 2003 004 545
- US-A1- 2009 138 042

## Description

### BACKGROUND OF THE INVENTION

### 1. Field of the Invention

The invention relates to a system for securing tissue to bone and more particularly to adjustable tensioning of tissue which eliminates the need for knot-tying by a user.

### 2. Description of the Related Art

A common injury, especially among athletes, is the complete or partial detachment of tendons, ligaments or other soft tissues from bone. Tissue detachment may occur during a fall, by overexertion, or for a variety of other reasons. Surgical intervention is often needed, particularly when tissue is completely detached from its associated bone. Currently available devices for tissue attachment include screws, staples, suture anchors and tacks. An example of a cannulated suture anchor is provided in U.S. Patent Application Publication No. 2008/0147063 by Cauldwell et al.

There are a number of suture implant systems which proclaim to be "knotless", that is, to not require a surgeon to tie a knot during surgery. Many such systems control tension on tissue by the depth to which an anchor is driven into bone. U.S. Patent Nos. 5,782,864 and 7,381,213 by Lizardi disclose certain types of suture anchors which capture a fixed-length loop of suture. Adjustable loop knotless anchor assemblies utilizing an anchor element inserted into a sleeve are described by Thal in U.S. Patent Nos. 5,569,306 and 6,045,574 and in U.S. Patent Application Publication No. 2009/0138042. This last document Reference describes a suture fixation system, in which hollow sleeve is embedded within bone and the inside of the sleeve is shaped to securely lock a separate anchor element within the sleeve. An adjustable suture loop that passes through tissue is captured by the anchor element and inserted into the sleeve. The suture loop includes a one-way sliding knot that allows the size of the suture loop element to be adjusted. After the anchor element is secured within the sleeve, the loop, capturing the tissue, is adjusted to the proper size to securely retain the tissue to a bone mass. The principle of operation of the anchor system is that tissue is captured by the collapsible loop, which is subsequently introduced into the proximal end of the anchor sleeve that has already been embedded in bone.

Suture anchor systems with sliding knots for repairing torn or damaged tissue, especially for meniscal repair, are disclosed in U.S. Patent No. 7,390,332 by Selvitelli et al. and are utilized in the OmniSpan™ meniscal repair system commercially available from DePuy Mitek Inc., 325 Paramount Drive, Raynham, Massachusetts 02767. Other suture anchor systems with sliding and locking knots for repairing tissue include U.S. Patent No. 6,767,037 by Wenstrom, Jr.

It is therefore desirable to adjust tension on a tissue after an anchor has been fixated in bone without requiring a surgeon to tie any knots, especially during arthroscopic procedures.

### SUMMARY OF THE INVENTION

A suture anchor system according to the present invention comprises a suture anchor, a collapsible suture loop affixed to the anchor and a tissue suture attached to the collapsible suture loop. The suture anchor has a central axial cannulation. The collapsible suture loop affixed to the anchor comprises a sliding knot. A post limb extends from the sliding knot, wherein tension upon the post limb collapses the loop. The tissue suture attached to the collapsible suture loop comprises a fixed tail extending from the sliding knot, wherein the collapsible loop and the post limb extend proximally out of the cannulation. A suture grasper is provided, adapted to be received through the cannulation to facilitate threading the tissue suture proximally through the cannulation after the tissue suture is loaded into soft tissue. The suture grasper comprises an elongated body passing through the cannulation with a suture capture mechanism distal of the suture anchor.

### BRIEF DESCRIPTION OF THE DRAWINGS

In what follows, a preferred embodiment of the invention is explained in more detail with reference to FIGs. 17 and 18A - 18H of the drawings, in which:
FIG. 1 is a perspective view of an adjustable anchor system having a closed loop and an adjustable loop of filament;
FIGS. 1A, 2 and 3 are schematic cross-sectional views of alternative anchor systems;
FIG. 4 is a schematic view of a closed loop after it has been pulled through a portion of tissue to be secured to bone, which may draw a portion of the adjustable loop with it through the tissue;
FIG. 5 illustrates an anchor being passed through the fixed loop and directed toward a hole formed in the bone;
FIG. 6 shows the anchor fixated in bone with tension to be applied on the post limb;
FIG. 7 illustrates the tissue in a desired position under final tension after the post limb has been trimmed;
FIG. 8 shows an alternative technique to that illustrated in FIGS. 6 and 7 in which the anchor engages the closed loop instead of passing through it;
FIGS. 8A-8C show yet other embodiments in which the closed loop has a sufficient length to extend from the anchor up to and through the tissue, as illustrated in enlarged view in FIG. 8B, and another, less-preferred embodiment in which the closed loop has been eliminated and the adjustable loop passes completely through tissue as illustrated in FIG. 8C;
FIG. 9 is a top plan view of a Tennessee Slider knot;
FIG. 10 is a front elevation view in cross-section of a further suture anchor system;
FIG. 11A is a perspective view of the suture anchor body of the suture anchor system of FIG. 10;
FIG. 11B is a top plan view of the suture anchor body of FIG. 11A;
FIG. 11C is a side elevation view of the suture anchor body of FIG. 11A;
FIG. 12 is a front elevation view in cross-section of a further suture anchor system;
FIGS. 13A to 13H are front elevation views in cross-section of a method of using the suture anchor system of FIG. 12;
FIG. 14 is a front elevation view in cross-section of a further suture anchor system, having a distal eyelet;
FIGS. 15A to 15E are front elevation views in cross-section of a method of using the suture anchor system of FIG. 14;
FIG. 16A is a front elevation view in cross-section of a further suture anchor system, having a distal eyelet and having a funnel shaped opening into the eyelet for loading suture into the eyelet;
FIG. 16B is a front elevation view in cross-section of a further suture anchor system, having a distal eyelet comprising overlapping arms for loading suture into the eyelet;
FIG. 16C is a front elevation view in cross-section of a further suture anchor system, having a forked distal tip for receiving suture;
FIG. 17 is a front elevation view in cross-section of an embodiment of a suture anchor system according to the present invention in which the sliding knot is proximal of the loop attachment to the anchor; and
FIGS. 18A to 18H are front elevation views in cross-section of an exemplary method of using the suture anchor system of FIG. 17.

### DETAILED DESCRIPTION OF THE PRESENTLY PREFERRED EMBODIMENTS

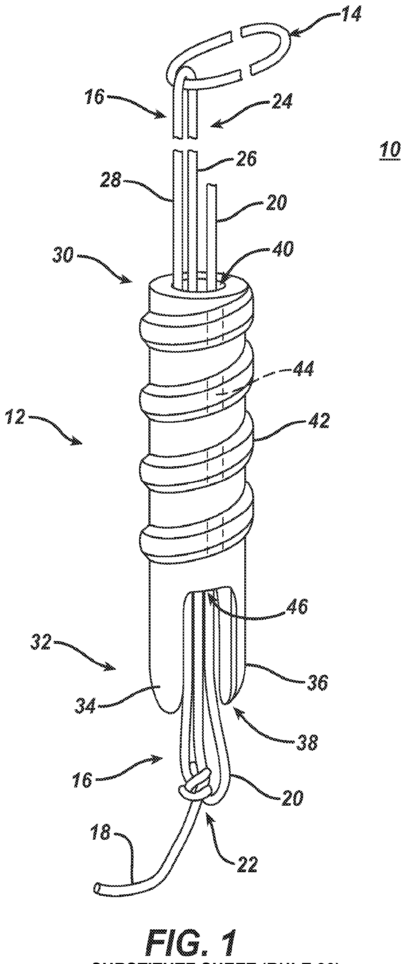

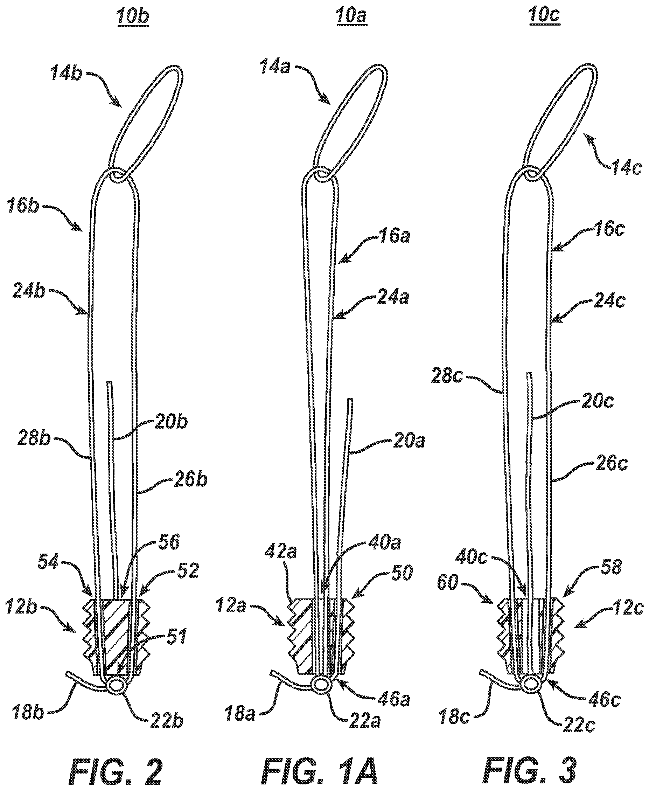

Adjustable anchor system 10, FIG. 1, has a suture anchor 12, a closed, fixed-length loop 14 of a first material, and a second filament 16 having a terminal end 18, a post limb 20, a sliding bunt line half hitch knot 22, and an adjustable loop 24 with loop limbs 26 and 28. In one construction, suture anchor 12 is similar to the cannulated suture anchor disclosed by Cauldwell et al. in U.S. Patent Application Publication No. 2008/0147063. In anchor systems according to the present invention, however, it is not necessary to have a post-like suture-engaging member or other occluding element over which one or more sutures or suture limbs pass to serve as a restriction to proximal movement; in many constructions, it is sufficient to have a restricted opening 46 to prevent withdrawal of knot 22 as discussed in more detail below, particularly in relation to FIGS. 1A-3.

Suture anchor 12 has a proximal end 30 and a distal end 32 with opposed distal arms 34 and 36 defining cut-out 38 between them. Passage 40 is an inner lumen which runs from proximal end 30 to distal cut-out 38. Although knot 22 is shown extending beyond cut-out 38 in FIG. 1 for purposes of illustration, knot 22 preferably is seated against restricted opening 46 between arms 34 and 36, or otherwise maintained at the distal end 32 by a cavity or other feature, during insertion of anchor system 10 into a patient to minimize interference by the knot 22 with the bone-engaging feature 42, or other exterior surface of anchor 12, and the bone in which suture anchor 12 is fixated.

One or more bone-engaging features 42, such as the helical thread illustrated in FIG. 1 or other features such as teeth, ridges, or other protrusions, are formed on the exterior of anchor 12 to enhance fixation in bone. In one construction, the suture anchor rotates to toggle into bone at its proximal end to minimize withdrawal. In a number of constructions, a hole is formed in bone prior to anchor insertion; in other constructions, a suture anchor is inserted directly into bone.

One or more passages or channels may be formed on the exterior of the suture anchor, such as channel 44 illustrated in phantom, FIG. 1, traversing bone-engaging element 42. Other configurations are illustrated in FIGS. 1A, 2 and 3 for adjustable anchor systems 10a, 10b and 10c, respectively, having first, fixed-length loops 14a, 14b, 14c and second, adjustable length filaments 16a, 16b, 16c, respectively. Anchor 12a, FIG. 1A, defines an inner lumen 40a and an external passage 50 extending from the distal end to the proximal end of anchor 12a. Sliding knot 22a, formed in second filament 16a, is seated against restricted opening 46a, adjustable loop 24a extends through passage 40a to capture closed loop 14a, and post limb 20a lies within external channel 50 in this construction.

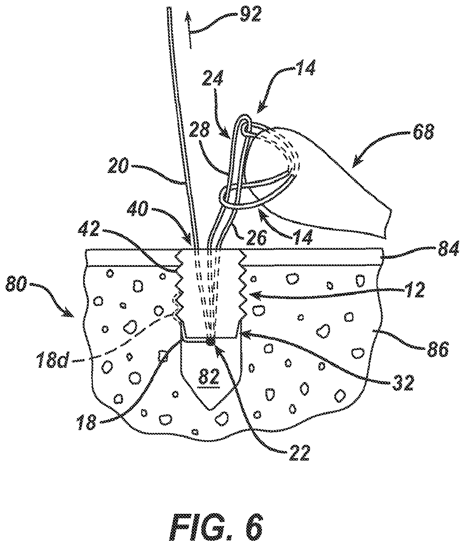

It is a matter of surgeon preference whether a terminal end, such as terminal end 18a, FIG. 1A, is kept at a length sufficient to lie against the exterior of at least one bone-engaging feature 42a to be trapped against bone during insertion, or is trimmed to a shorter length. Different examples of terminal end length are provided in FIGS. 6-8 below. Further, a restriction such as restricted opening 46a may be defined at least in part by engagement with bone when anchor 12a is fixated in bone to prevent knot 22a from moving with post limb 20a when tension is applied to post limb 20a as described in more detail below for procedures of using an anchor system.

Anchor system 10b, FIG. 2, has at least three external passages or channels 52, 54 and 56 without any internal passages in this construction. Knot 22b is maintained at the distal end of anchor 12b by occlusion 51, defined at least in part by the distal surface of anchor 12b, while limbs 26b, 28b of loop 24b lie within passages 52, 54 and post limb 20b of second filament 16b lies within passage 56. As described above, occlusion 51 may be defined in part by engagement of anchor 12b with bone after fixation.

Anchor system 10c, FIG. 3, has an internal passage 40c through which post limb 20c extends from a restricted opening 46c which holds knot 22c. External passages 58, 60 carry limbs 26c, 28c of adjustable loop 16c. Although anchors 12a, 12b and 12c are shown without distal-extending arms in those constructions, in other constructions one or more such distal extensions or other protrusions are provided, similar in some constructions to Cauldwell et al. cited above or to U.S. Patent No. 7,381,213 by Lizardi. In yet other constructions, a cylindrical or otherwise circumferential cavity, bowl or countersink feature is provided at the distal end of the anchor to seat the knot 22 during insertion and fixation.

In preferred constructions, loop 14, also referred to as a first filament, and second filament 16 are formed of one or more types of sutures. Acceptable diameters for second filament 16 include size 0 or size 2 suture, such as Orthocord™ suture commercially available from DePuy Mitek, while the same or larger diameters such as size 2 to size 5 suture are preferred for loop 14, such as Ethibond™ suture available from Ethicon. Orthocord™ suture is approximately fifty-five to sixty-five percent PDS™ polydioxanone, which is bioabsorbable, and the remaining percent ultra high molecular weight polyethylene, while Ethibond™ suture is primarily high strength polyester. In some constructions, especially for shoulder repair procedures, loop 14 has a fixed length of approximately 25.4 mm (one inch) while adjustable loop 24 has a length of at least 457,2 mm (eighteen inches). The amount and type of bioabsorbable material, if any, utilized in the first or second filament is primarily a matter of surgeon preference for the particular surgical procedure to be performed.

While the same type of suture can be used for both loop 14 and filament 16, a suture having a lower abrasive property at its surface is preferred for the first material forming closed loop 14. The lower abrasive property can be achieved by a larger diameter, a softer composition, a softer braid, plait or strand pattern, or a combination of such characteristics. In some constructions, the suture material for closed loop 14 is tied with a fixed knot to form the fixed-length loop 14. In other constructions, loop 14 is molded or otherwise formed as a ring of material.

Slidable knot 22 has been described as a bunt line half hitch knot in some constructions, but other suitable knots will be readily apparent to those of ordinary skill in the suture tying art. The term "slidable" as used herein is intended to include slidable, lockable knots as well as slidable knots. Several types of suitable knots are described in the Arthroscopic Knot Tying Manual (2005) available from DePuy Mitek, as well as in U.S. Patent No. 6,767,037 by Wenstrom, Jr.

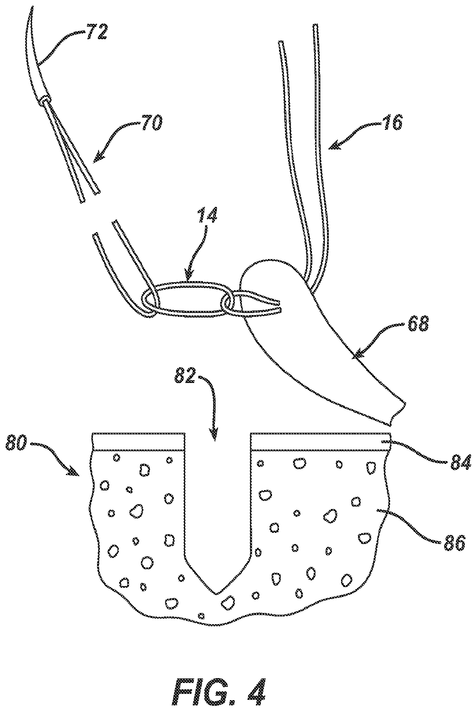

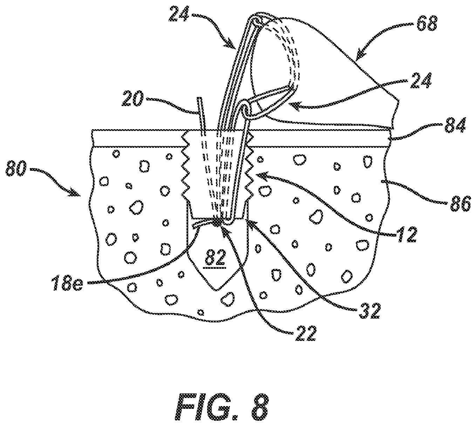

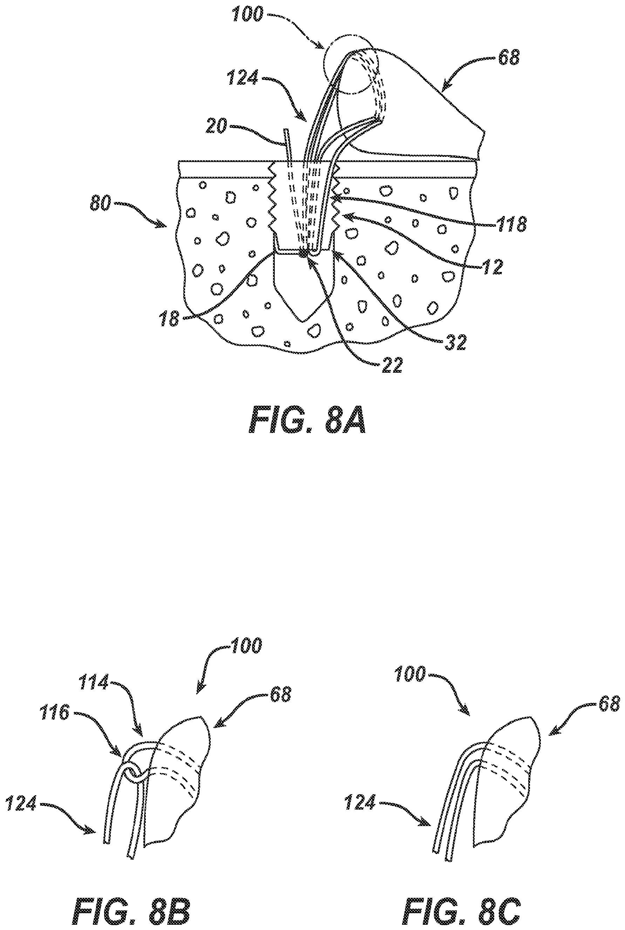

One procedure for utilizing a cannulated anchor system similar to that shown in FIG. 1 is illustrated in FIGS. 4-7 for attaching tissue 68 to bone 80. Reference numerals utilized to describe the system shown for this procedure follow the numerals utilized for system 10, FIG. 1, for simplicity and clarity, although a number of other types of anchors with different filament limb arrangements as illustrated in other Figures could also be utilized in a similar manner. An initial suture 70, FIG. 4, having a needle 72 at its distal end is passed through tissue 68 to draw at least closed loop 14 at least partially through tissue 68. Alternatively, a suture passing instrument is inserted through tissue 68 to grasp the closed loop 14 and pull it through the tissue 68. The extent to which elongated, adjustable loop 24 is drawn through tissue 68, and whether an anchor passes through or engages the closed loop 14 or adjustable loop 24, are described in more detail below relative to FIGS. 8-8C.

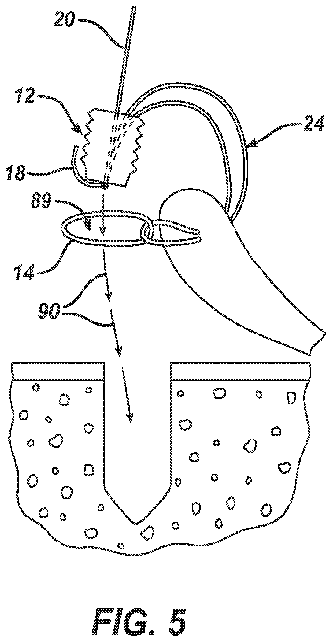

In this procedure, a hole 82, FIG. 4, is formed through compact layer 84 into cancellous layer 86 of bone 80 at a desired repair location. Anchor 12, FIG. 5, is passed through an opening 89 in closed loop 14 as indicated by arrows 90 and is fixated in bone as shown in FIG. 6. Preferably, post limb 20 is extracted from the closed loop 14 after anchor 12 passes through the opening 89 in closed loop 14, FIG. 5, so that post limb 20 can pull directly one of adjustable loop limbs through the knot 22 without being constrained by closed loop 14. Terminal end 18 is trapped between bone 86 and a portion of distal end 32 of anchor 12 in this construction, and sliding knot 22 is held by a restricted opening in internal lumen 40. Alternatively, terminal end 18 has a sufficient length so that it extends proximally along the exterior of the anchor 12 past a plurality of bone engaging features 42 as illustrated in phantom as length 18d, or is a shorter length 18e as shown in FIG. 8.

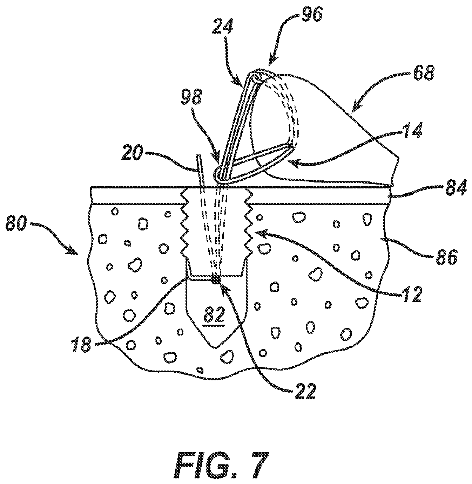

After fixation of anchor 12, FIG. 6, proximal tension is applied to post limb 20 as indicated by arrow 92. As post limb 20 is moved proximally, adjustable loop 24 readily slides through closed loop 14 as limbs 26 and 28 are shortened. Tissue 68 is thereby drawn toward anchor 12 until a final desired position, under desired tension, is achieved as shown in FIG. 7. Closed loop 14 engages the elongated loop 24 at first and second locations 96 and 98.

Other systems and methods are shown in FIGS. 8-8C as alternatives to the final configuration shown in FIG. 7. Terminal end 18e, FIG. 8, is intentionally short so that it is not trapped between the anchor 12 and bone 80. In this construction, terminal end 18e remains within the bone hole 82 and is not placed under tension of any type.

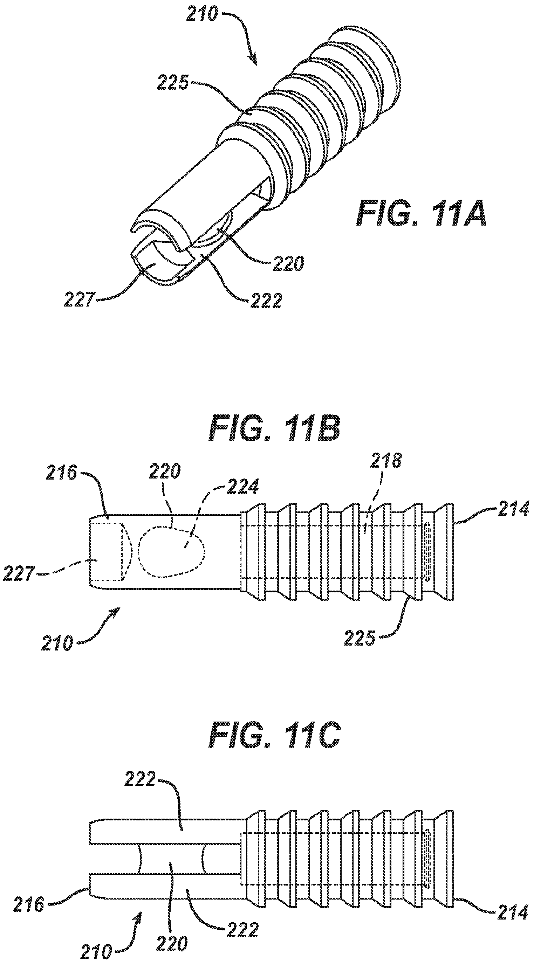

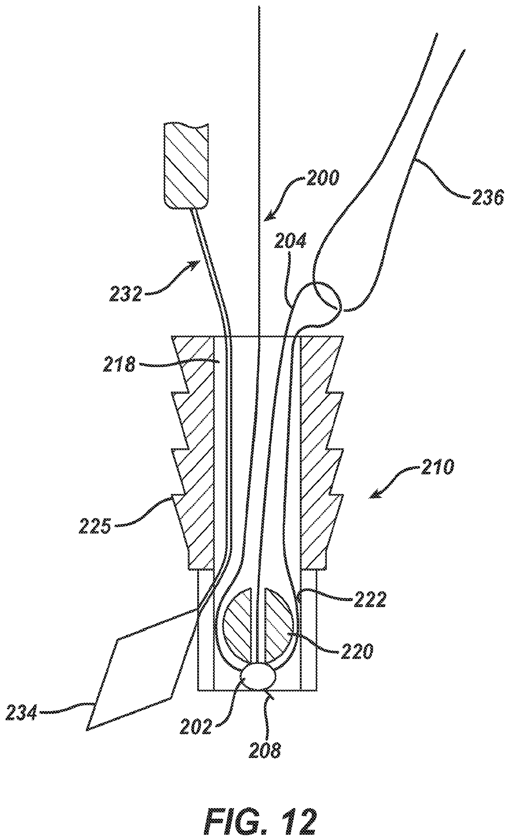

Instead of requiring an opening 89, FIG. 5, in closed loop 14 to be greater than the circumference of the anchor 12 so that anchor 12 can pass completely through the closed loop 14, in other constructions the distal end 32 engages a portion of the closed loop 14 as shown in FIG. 8. Filament engagement can be accomplished such as shown in FIGS. 11 and 12 of U.S. Patent No. 7,381,213 by Lizardi. However, one benefit achieved is that further tensioning of tissue 68 is possible after anchor fixation by pulling on the post limb of the adjustable loop. Another benefit is that the anchor inserter or driver and related driver instruments are removed prior to final tensioning and positioning of the tissue to be repaired to provide improved visual and tactile feedback to the surgeon.

Other arrangements of filaments are illustrated in FIGS. 8A-8C. Anchor 12, FIG. 8A, engages filament portion 118 which passes through tissue 68 to emerge at the other, proximal side in the circled region indicated at 100. In the construction shown in FIG. 8B for that circled region 100, adjustable loop 124 passes through a portion of closed loop 114. In other words, the same filament limbs form portions 114 and 118 of a single closed, fixed-length loop. This arrangement is preferred because the limbs of adjustable loop 124 are able to slide over the closed loop filament at location 116 without passing through tissue as adjustable loop 124 is reduced in size.

In contrast, limbs of adjustable portion 124 pass through tissue 68 in FIG. 8C, which represents an alternative configuration for FIG. 8A, and the adjustable limbs emerge to form portion 118 which is engaged by the distal end 32 of anchor 12. In other words, no closed loop is utilized in the configuration represented by FIG. 8C. However, eliminating the fixed-length loop is less desirable because the adjustable loop may tend to lock on itself, and may bind with or cause damage to the soft tissue through which it passes. The adjustable loop may be prone to locking on itself even if the anchor is passed completely through the adjustable loop. Further, adjustability of portion 118, FIG. 8C, may be further impeded by an interference fit with bone, unless the limbs of portion 118 are properly aligned in channels or other exterior passages along anchor 12.

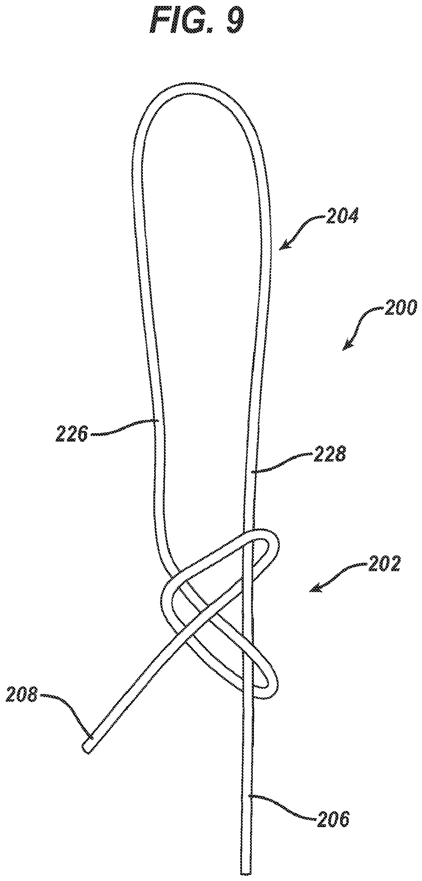

A suture 200 can employ a Tennessee Slider knot 202 (FIG. 9) in substitution for the blunt line half hitch knot 22. It comprises a loop 204, post limb 206 and terminal end 208. The terminal end 208 passes over and then behind the post limb 206, then over the loop 204, and then finally behind the post limb 206 and back out.

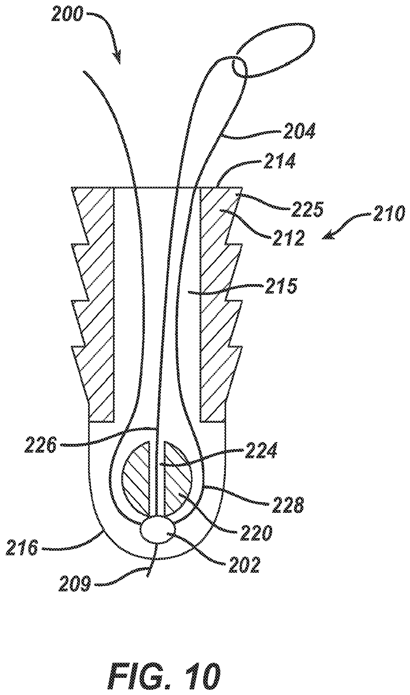

FIGS. 10 and 11A to 11C illustrate a further suture anchor 210. It comprises a body 212 having a proximal end 214 and distal end 216 with an axial lumen 218 passing through the proximal end 214. The distal end 216 is slotted with a saddle 220 between two distal sides 222. An aperture 224 passes axially through the saddle 220. Annular barbed flanges 225 about the body 212 provide for enhanced fixation.

The suture 200 with the Tennessee Slider knot 202 is positioned in the anchor 210 with the knot 202 distal of the aperture 224 in a pocket 227 created into the sides 222, a fixed tail 226 of the loop 204 passing proximally from the knot 202 through the aperture 224 and an adjustable tail 228 of the loop 204 wrapping around the saddle 220 to meet the knot 202. The loop 204 extends proximally out of the lumen 218. The post limb 206 extends proximally around the opposite side of the saddle 220 from the adjustable tail 228 and also extends proximally out of the lumen 218. The anchor 210 can be employed in a fashion similar to the aforementioned embodiments.

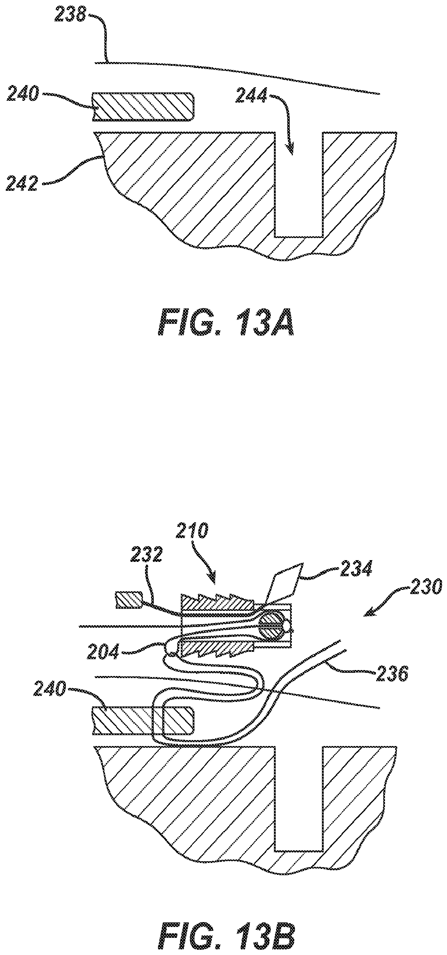

FIGS. 12 and 13A to 13G illustrate an alternative procedure. An anchor system 230 comprises the suture anchor 210 with suture 200 rigged as illustrated in FIG. 10 and with a flexible wire suture capture device 232 having a suture capture loop 234 (such as a Chia Percpasser available from DePuy Mitek, Inc. of Raynham, MA) threaded through the lumen 218 with the suture capture loop 234 distal of the anchor body 212. A separate length of tissue suture 236 is threaded through the loop 204.

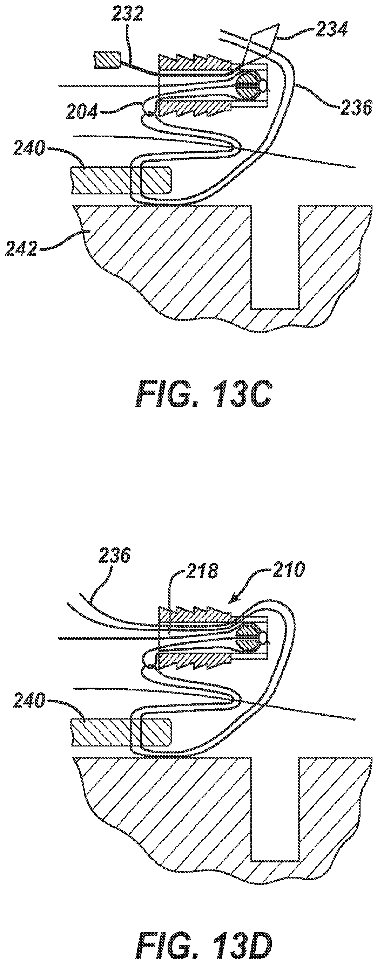

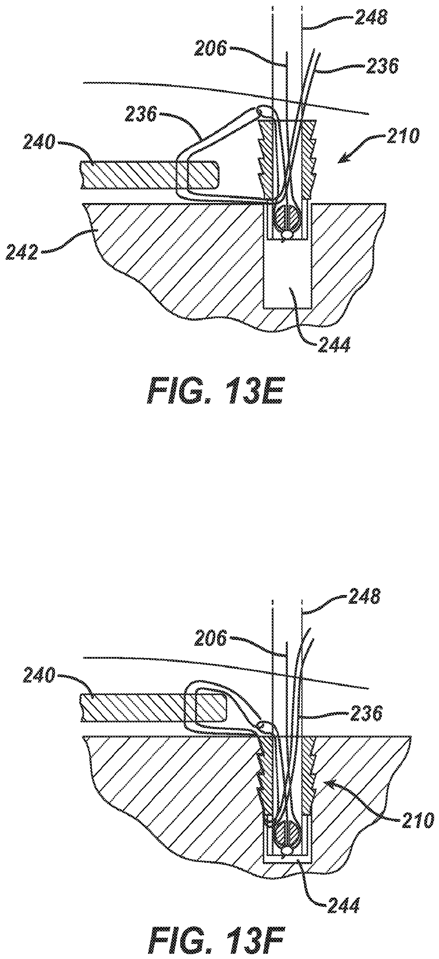

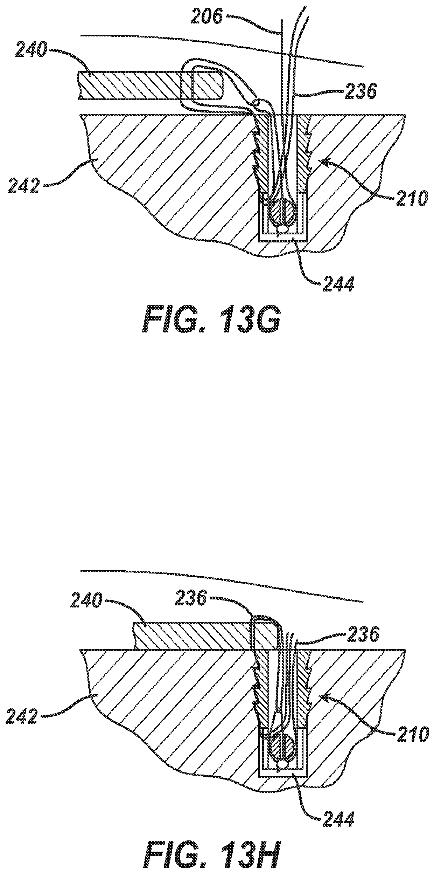

Typically the anchor system 230 is employed arthroscopically through a cannula which would pass through a patient's skin 238 to the tissue 240 and bone 242 where a repair is to be effected, but is not here illustrated to better focus on the rigging and motion of the sutures 200 and 236, and suture anchor 210. First a bone hole 244 is created adjacent the tissue 240 (FIG. 13A). The tissue suture 236 is passed through the tissue 240 (such as a rotator cuff tendon) and pulled back out of the cannula (not shown) to be outside of the skin 238 (FIG. 13B) where it can be threaded through the suture capture loop 234 (FIG. 13C). A suture passer for passing suture through tissue such as the EXPRESSEW Flexible Suture Passer available from DePuy Mitek, Inc. of Raynham, MA can be employed for this. The suture capture device 232 is pulled proximally out of the lumen 218 threading the tissue suture 236 through the lumen 218 (FIG. 13D). The suture anchor 210 is then passed down the cannula (not shown) to the bone hole 244. The suture anchor distal end 216 is placed into the hole 244 and slack is taken out of the tissue suture 236 (FIG. 13E). The anchor 210 is then driven into the bone hole 244 thus locking the tissue suture 236 between the anchor 210 and the bone 242 (FIG. 13F). Preferably the anchor 210 is inserted and driven into the bone hole 244 via a cannulated driver 248 with the post limb 206 situated within a cannulation 250 through the driver 248. After the anchor 210 is driven into the bone hole 244 the driver 248 is preferably removed (FIG. 13G) and then the post limb 206 is pulled to collapse the loop 204 and tension the tissue suture 236 (FIG. 13H).

Having the combination of the collapsible loop 204 and the tissue suture 236 provides great advantage while collapsing the loop 204 during tensioning of the repair. The loop 204 slides over the tissue suture 236 rather than sliding through the tissue itself which allows for easier tensioning of the repair as the coefficient of friction between the loop 204 and the tissue suture 236 will be less than if the loop 204 were sliding through the tissue itself and it minimizes any effects upon the tissue.

Although shown with a single anchor 210 a typical tissue repair might involve a row of anchors or multiple rows of anchors. The anchor 210 and other embodiments are for instance useful with dual row rotator cuff repairs in which a first medial row of anchors is placed beneath the cuff with suture threrefrom extending up through the cuff and running to a second lateral row of anchors located near the edge of the cuff. The suture extending up through the cuff from the medial row could replace the tissue suture 236 and be captured by anchors 210, in which case the suture capture device would preferably rigged through the lumen 218 as in FIG. 10 and then back through the loop 204 so that it would pass the suture from the medial anchors in place of the tissue suture 236. To facilitate loading such a loop of tissue suture the collapsing loop could be rigged with a closed loop such as loop 14 with the anchor being passed therethrough and through the tissue suture loop so that the collapsing loop rides against the closed loop as it collapses.

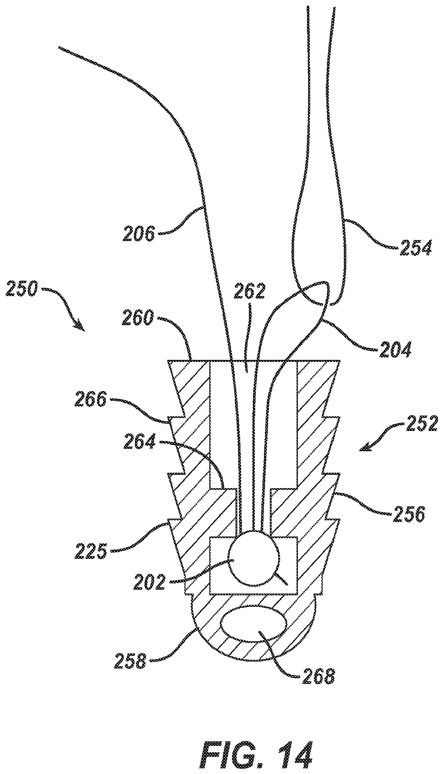

FIG. 14 illustrates a further anchor system 250. It comprises a suture anchor 252 with suture 200 and a tissue suture 254. The anchor 252 comprises a body 256 having a distal end 258 and proximal end 260. An axial lumen 262 enters from the proximal end 260 and has a restriction 264 therein. Barbed flanges 266 encircle the body 256. An eyelet 268 is provided at the distal end 258. The suture 200 is situated in the anchor with the knot 202 distal of the restriction 264 and the loop 204 and post limb 206 passing proximally through the restriction 264 and out of the lumen 262. The restriction 264 restrains the knot 202.

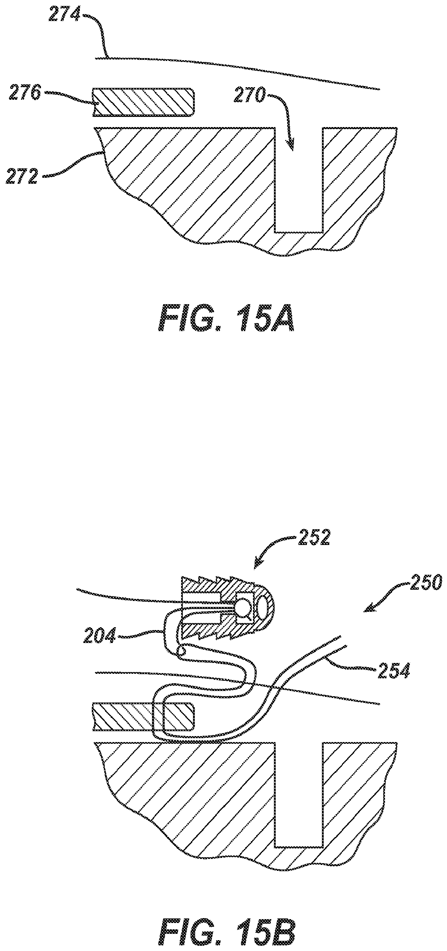

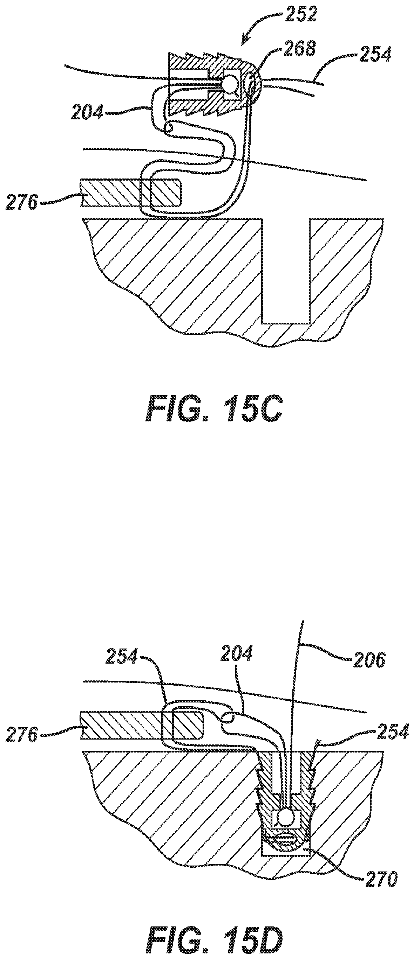

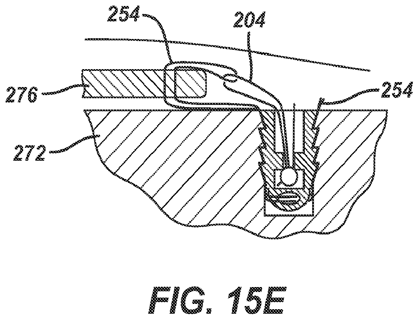

FIGS. 15A to 15E illustrate a procedure for the anchor system 250, and as in previous depictions the arthroscopy cannula and instrumentation are omitted for clarity. A bone hole 270 is formed in a bone 272 under a patient's skin 274 adjacent a tissue 276 (FIG. 15A). The tissue suture 254 is passed through the tissue (FIG. 15B) and passed through the eyelet 268 (FIG. 15C). A suture capture device 232 (not shown in FIGS. 15A TO 15E) could be employed through the eyelet 268 to ease loading the suture 254 therethrough. The anchor 252 is placed at the bone hole 270, the slack is removed from the tissue suture 254 and then the anchor is driven into the bone hole 270 to lock the tissue suture between the anchor 252 and the bone 272, preferably on two sides of the anchor 252. Tension applied to the post limb 206 collapses the loop 204 and tensions the tissue suture 254.

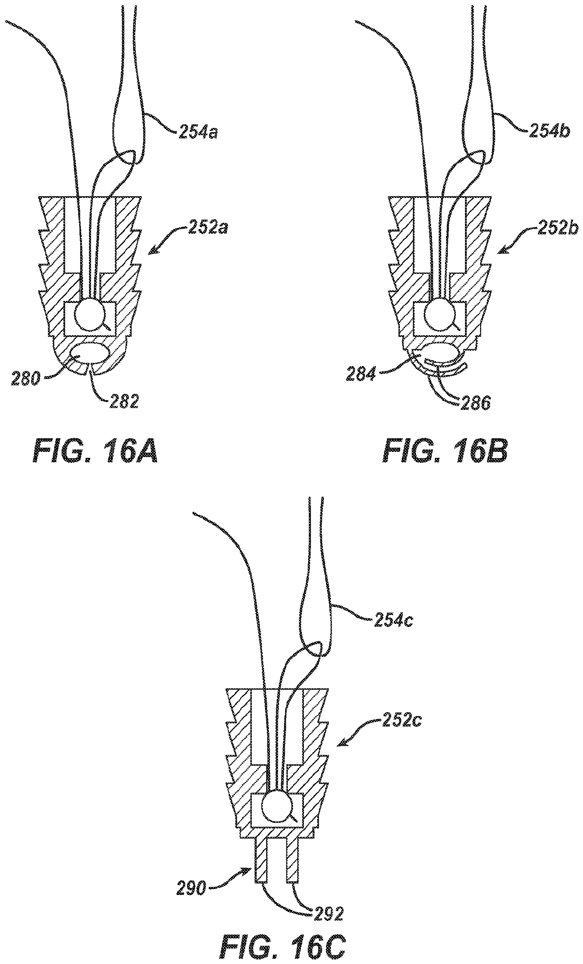

FIGS. 16A to 16C illustrate variations of the suture anchor system 250. Like parts in anchors 252a, 252b and 252c have like part numbers to the anchor 250 with the exception of their subscripts. Anchor 252a (FIG. 16A) has an eyelet 280 having a funnel shaped slot 282 entry therein for loading the tissue suture 254a into the eyelet and then inhibiting its passage back out of the slot 282. Anchor 252b (FIG. 16B) has an eyelet 284 formed of overlapping arms 286 creating a tortuous path 288 into the eyelet 284 for the tissue suture 254b. Anchor 252c (FIG. 16C) has a fork tip 290 formed of distally projecting tines 292 between which the tissue suture 254c can be captured.

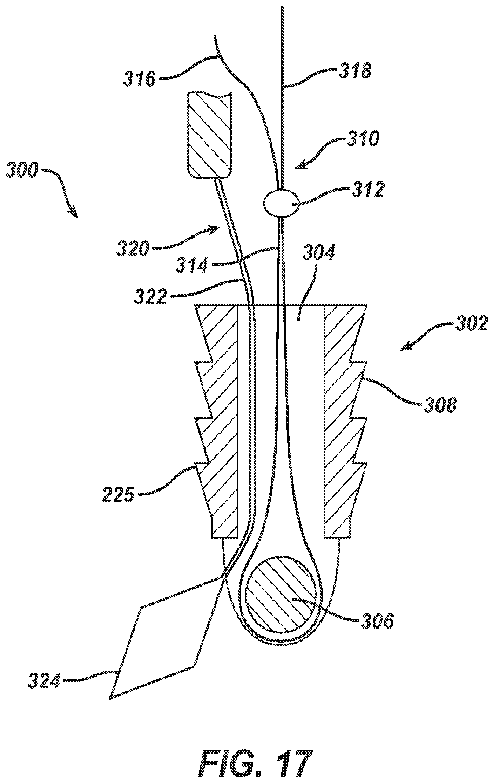

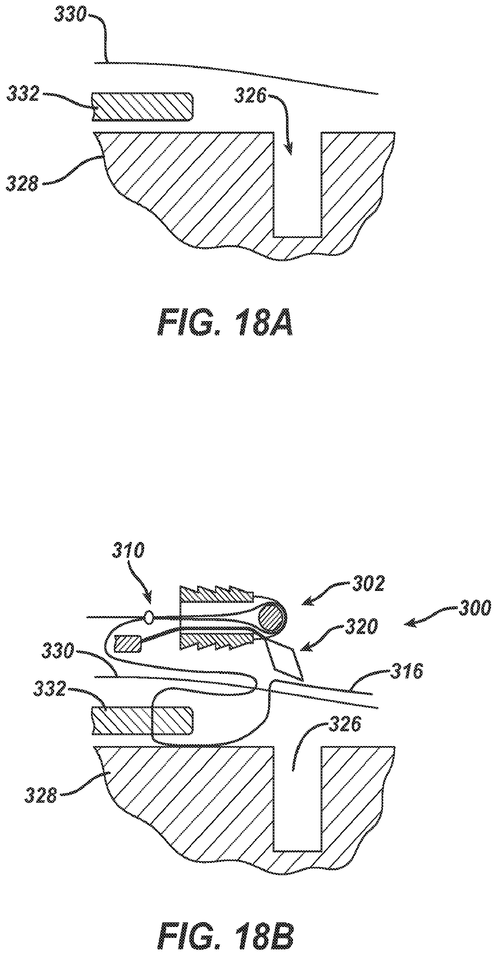

FIGS. 17 and 18A to 18H illustrate an embodiment of a suture anchor system 300 according to the present invention. It comprises a suture anchor 302 having an elongated cylindrical shape with an axial cannulation 304 and internal suture saddle 306. Annular barb-shaped flanges 308 encircle the anchor 302 to enhance its bone fixation. A suture 310 comprise a sliding knot 312 forming a collapsing loop 314 around the suture saddle 306 with a long fixed tail 316 and collapsing tail 318. A suture threader 320 comprises an elongated flexible threading wire 322 received through the cannulation 304 and terminating in a distal suture capture loop 324 distal of the anchor 302.

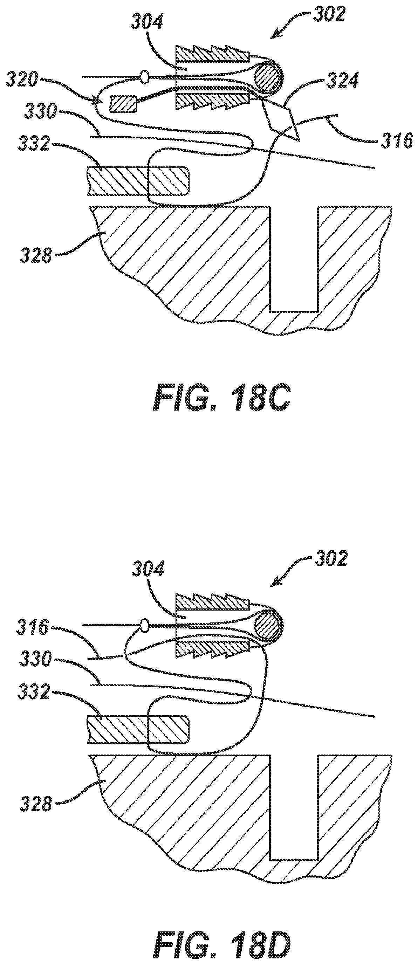

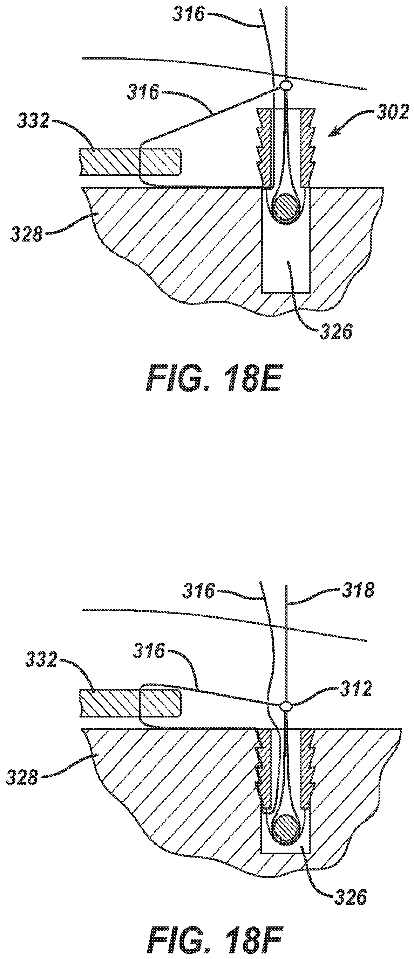

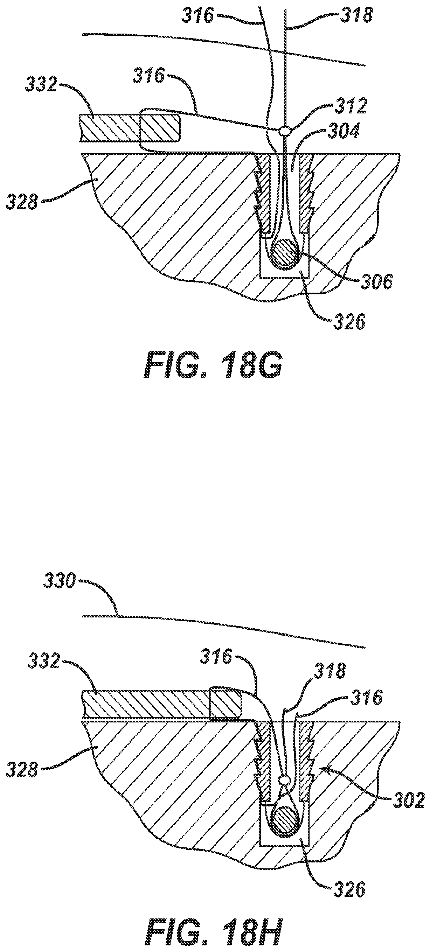

To employ the suture anchor system 300, a bone hole 326 is formed in a bone 328 beneath a patient's skin 330 and adjacent a soft tissue 332, such as for example a tendon, (FIG. 18A) which is to be attached to the bone 328 with the suture anchor system 300. The fixed tail 316 is passed through the soft tissue 332 and brought back outside of the skin 330 (FIG. 18B). As in the prior procedure descriptions the procedure is preferably performed arthroscopically and the associated arthroscopy equipment and the anchor driver are omitted from the FIGS. for clarity in illustrating the operation of the suture anchor system 300. The fixed tail 316 is loaded into the suture capture loop 324 (FIG. 18C) and the suture threader 320 is withdrawn proximally through the cannulation 304 to load the fixed tail 316 through the cannulation 304 (FIG. 18D). The suture anchor 302 is then placed into the opening of the bone hole 326 and the slack is taken out of the fixed tail 316 (FIG. 18E). When the suture anchor 302 is driven into the bone hole 326 it traps the fixed tail 316 between the anchor 302 and the bone 328 (FIG. 18F). By pulling on the collapsing tail 318 the collapsing knot 312 collapses the loop 304 and moves down toward the suture saddle 306 thereby drawing fixed tail 316 and thereby the soft tissue 332 toward the anchor 302 (FIG. 18G). The collapsing tail 318 and fixed tail 316 can then be trimmed completing the procedure (FIG. 18H). The resulting knot is very strong as tension between the fixed tail 316 where it leaves the knot 312 and the loop 314 cinches the knot and such tension is provided by the load between the soft tissue 332 and the suture saddle 306 thus providing a strong fixation.

The suture anchors described may be made from a number of suitable materials including a metallic material, a non-biodegradable polymer, a biodegradable polymer, or a composite of a biodegradable polymer or copolymer and a bioceramic. The term biodegradable as used herein is defined to mean materials that degrade in the body and then are either absorbed into or excreted from the body. The term bioceramic as defined herein is defined to mean ceramic and glass materials that are compatible with body tissue. The bioceramics are preferably biodegradable.

The metallic materials that can be used to manufacture the anchors described include stainless steel, titanium, alloys of nickel and titanium, or other biocompatible metallic materials.

The non-biodegradable materials that can be used to manufacture the anchors described include polyethylene, polypropylene, PEEK (polyetheretherketone), or other biocompatible non-absorbable polymers.

The biodegradable polymers that can be used to manufacture the anchors described include biodegradable polymers selected from the group consisting of aliphatic polyesters, polyorthoesters, polyanhydrides, polycarbonates, polyurethanes, polyamides and polyalkylene oxides. Preferably, the biodegradable polymers are aliphatic polyester polymers and copolymers, and blends thereof. The aliphatic polyesters are typically synthesized in a ring opening polymerization. Suitable monomers include but are not limited to lactic acid, lactide (including L-, D-, meso and D,L mixtures), glycolic acid, glycolide, .epsilon.-caprolactone, p-dioxanone (1,4-dioxan-2-one), trimethylene carbonate (1,3-dioxan-2-one), .delta.-valerolactone, and combinations thereof.

The bioceramics that can be used in the composite anchors described include ceramics comprising mono-, di-, tri-, .alpha.-tri-, .beta.-tri-, and tetra-calcium phosphate, hydroxyapatite, calcium sulfates, calcium oxides, calcium carbonates, magnesium calcium phosphates. It is particularly preferred to use a .beta.-tritricalcium phosphate. In addition to bioceramics, bioglasses may also be used in the composite screws. The bioglasses may include phosphate glasses and bioglasses.

Suitable biocompatible synthetic polymers can include polymers selected from the group consisting of aliphatic polyesters, poly(amino acids), copoly(ether-esters), polyalkylene oxalates, polyamides, tyrosine derived polycarbonates, poly(iminocarbonates), polyorthoesters, polyoxaesters, polyamidoesters, polyoxaesters containing amine groups, poly(anhydrides), polyphosphazenes, polyurethanes, poly(ether urethanes), poly(ester urethanes), poly(propylene fumarate), poly(hydroxyalkanoate) and blends thereof.

Aliphatic polyesters include, but are not limited to, homopolymers and copolymers of lactide (which includes lactic acid, D-,L- and meso lactide); glycolide (including glycolic acid); .epsilon.-caprolactone; p-dioxanone (1,4-dioxan-2-one); trimethylene carbonate (1,3-dioxan-2-one); alkyl derivatives of trimethylene carbonate; .delta.-valerolactone; .beta.-butyrolactone; .gamma.-butyrolactone; .epsilon.-decalactone; hydroxybutyrate; hydroxyvalerate; 1,4-dioxepan-2-one (including its dimer 1,5,8,12-tetraoxacyclotetradecane-7,14-dione); 1,5-dioxepan-2-one; 6,6-dimethyl-1,4-dioxan-2-one; 2,5-diketomorpholine; pivalolactone; .alpha.,.alpha. diethylpropiolactone; ethylene carbonate; ethylene oxalate; 3-methyl-1,4-dioxane-2,5-dione; 3,3-diethyl-1,4-dioxan-2,5-dione- ; 6,6-dimethyl-dioxepan-2-one; 6,8-dioxabicycloctane-7-one and polymer blends thereof. Additional exemplary polymer or polymer blends include, by non-limiting example, a polydioxanone, a polyhydroxybutyrate-co-hydrox- yvalerate, polyorthocarbonate, a polyaminocarbonate, and a polytrimethylene carbonate. Aliphatic polyesters can be homopolymers or copolymers (random, block, segmented, tapered blocks, graft, triblock, etc.) having a linear, branched or star structure. Poly(iminocarbonates) are understood to include those polymers as described by Kemnitzer and Kohn, in the Handbook of Biodegradable Polymers, edited by Domb, et. al., Hardwood Academic Press, pp. 251-272 (1997). Copoly(ether-esters) are understood to include those copolyester-ethers as described in the Journal of Biomaterials Research, Vol. 22, pages 993-1009, 1988 by Cohn and Younes, and in Polymer Preprints (ACS Division of Polymer Chemistry), Vol. 30(1), page 498, 1989 by Cohn (e.g., PEO/PLA). Polyalkylene oxalates include those described in U.S. Pat. Nos. 4,208,511; 4,141,087; 4,130,639; 4,140,678; 4,105,034; and 4,205,399. Polyphosphazenes, co-, ter- and higher order mixed monomer based polymers made from L-lactide, D,L-lactide, lactic acid, glycolide, glycolic acid, para-dioxanone, trimethylene carbonate and E-caprolactone such as are described by Allcock in The Encyclopedia of Polymer Science, Vol. 13, pages 31-41, Wiley Intersciences, John Wiley & Sons, 1988 and by Vandorpe, et al in the Handbook of Biodegradable Polymers, edited by Domb, et al., Hardwood Academic Press, pp. 161-182 (1997). Polyanhydrides include those derived from diacids of the form HOOC--C.sub.6H.sub.4--O--(-CH.sub.2).sub.m--O--C.sub.6H.sub.4--COOH, where "m" is an integer in the range of from 2 to 8, and copolymers thereof with aliphatic alpha-omega diacids of up to 12 carbons. Polyoxaesters, polyoxaamides and polyoxaesters containing amines and/or amido groups are described in one or more of the following U.S. Pat. Nos. 5,464,929; 5,595,751; 5,597,579; 5,607,687; 5,618,552; 5,620,698; 5,645,850; 5,648,088; 5,698,213; 5,700,583; and 5,859,150. Polyorthoesters such as those described by Heller in Handbook of Biodegradable Polymers, edited by Domb, et al., Hardwood Academic Press, pp. 99-118 (1997).

## Claims

1. A suture anchor system (300) comprising:
a suture anchor (302) having a central axial cannulation (304);
a collapsible suture loop (314) affixed to the anchor (302) and comprising a sliding knot (312); and
a post limb (318) extending from the sliding knot (312) wherein tension upon the post limb (318) collapses the loop (314);
**characterized by** a tissue suture attached to the collapsible suture loop (314) and comprising a fixed tail (316) extending from the sliding knot (312), wherein the collapsible loop (314) and the post limb (318) extend proximally out of the cannulation (304); and
a suture grasper (320) adapted to be received through the cannulation (304) to facilitate threading the tissue suture proximally through the cannulation (304) after the tissue suture is loaded into soft tissue (332), wherein the suture grasper (320) comprises an elongated body (322) passing through the cannulation (304) with a suture capture mechanism (324) distal of the suture anchor (302).

2. The suture anchor system (300) of claim 1 wherein an attachment member (306) on the anchor (302) passes through the collapsible suture loop (314) to affix the collapsible loop (314) to the anchor (302).

3. The suture anchor system (300) of claim 2 wherein the attachment member is a post (306) in the anchor (302).

4. The suture anchor system (300) according to claim 3 wherein the sliding knot (312) is disposed proximal of the post (306).

5. The suture anchor system (300) of claim 1 and further comprising bone engaging protrusions (308) about the suture anchor (302).

6. The suture anchor system (300) of claim 1 wherein the suture anchor (302), collapsible loop (314) and tissue suture are sterile and packaged in a bacteria proof enclosure.

7. The suture anchor system (300) of claim 1 wherein the anchor (302) has an elongated cylindrical shape whereby to be fixedly received within a hole (326) drilled into a bone (328).

8. The suture anchor system (300) of claim 1 wherein the suture capture mechanism (324) is a loop of flexible material through which the tissue suture (316) can be threaded.

## Patentansprüche

1. Nahtmaterialankersystem (300), umfassend:
einen Nahtmaterialanker (302) mit einer mittleren axialen Kanülierung (304),
eine zusammenfaltbare Nahtmaterialschlaufe (314), die an dem Anker (302) befestigt ist und einen Gleitknoten (312) umfasst, und
ein Stützglied (318), das sich von dem Gleitknoten (312) erstreckt, wobei sich die Schlaufe (314) bei Spannung auf das Stützglied (318) zusammenfaltet,
**gekennzeichnet durch** ein Gewebenahtmaterial, das an der zusammenfaltbaren Nahtmaterialschlaufe (314) angebracht ist und einen festgelegten Schwanz (316) umfasst, der sich von dem Gleitknoten (312) erstreckt, wobei sich die zusammenfaltbare Schlaufe (314) und das Stützglied (318) proximal aus der Kanülierung (304) erstrecken, und
einen Nahtmaterialgreifer (320), der dazu ausgeführt ist, durch die Kanülierung (304) aufgenommen zu werden, um das Einfädeln des Gewebenahtmaterials proximal durch die Kanülierung (304) zu erleichtern, nachdem das Gewebenahtmaterial in Weichgewebe (332) eingebracht worden ist, wobei der Nahtmaterialgreifer (320) einen länglichen Körper (322) umfasst, der durch die Kanülierung (304) geht, wobei ein Nahtmaterialgreifmechanismus (324) distal von dem Nahtmaterialanker (302) liegt.

2. Nahtmaterialankersystem (300) nach Anspruch 1, wobei ein Anbringglied (306) an dem Anker (302) durch die zusammenfaltbare Nahtmaterialschlaufe (314) geht, um die zusammenfaltbare Schlaufe (314) an dem Anker (302) zu befestigen.

3. Nahtmaterialankersystem (300) nach Anspruch 2, wobei das Anbringglied eine Stütze (306) in dem Anker (302) ist.

4. Nahtmaterialankersystem (300) nach Anspruch 3, wobei der Gleitknoten (312) proximal zu der Stütze (306) angeordnet ist.

5. Nahtmaterialankersystem (300) nach Anspruch 1, ferner umfassend Knocheneingriffsvorsprünge (308) um den Nahtmaterialanker (302) herum.

6. Nahtmaterialankersystem (300) nach Anspruch 1, wobei der Nahtmaterialanker (302), die zusammenfaltbare Schlaufe (314) und das Gewebenahtmaterial steril und in einer bakteriendichten Umhüllung verpackt sind.

7. Nahtmaterialankersystem (300) nach Anspruch 1, wobei der Anker (302) eine längliche zylindrische Form hat, dank derer er fest in einem in einen Knochen (328) gebohrtes Loch (326) aufgenommen wird.

8. Nahtmaterialankersystem (300) nach Anspruch 1, wobei der Nahtmaterialgreifmechanismus (324) eine Schlaufe aus einem flexiblen Material ist, durch die das Gewebenahtmaterial (316) gefädelt werden kann.

## Revendications

1. Système d'ancrage (300), comprenant :
un ancrage de suture (302) ayant une canulation axiale centrale (304) ;
une boucle de suture affaissable (314) fixée à l'ancrage (302) et comprenant un nœud coulant (312) ; et
une branche de montant (318) s'étendant depuis le nœud coulant (312), une tension appliquée à la branche de montant (318) affaissant la boucle (314) ;
**caractérisé par** une suture de tissu attachée à la boucle de suture affaissable (314) et comprenant une queue fixe (316) s'étendant depuis le nœud coulissant (312), la boucle affaissable (314) et la branche de montant (318) s'étendant dans la direction proximale hors de la canulation (304) ; et
un dispositif de préhension de suture (320) prévu pour être reçu à travers la canulation (304) pour faciliter l'enfilement de la suture de tissu dans la direction proximale à travers la canulation (304) après que la suture de tissu a été chargée dans des tissus mous (332), le dispositif de préhension de suture (320) comprenant un corps allongé (322) passant à travers la canulation (304) avec un mécanisme de capture de suture (324) distal par rapport à l'ancrage de suture (302).

2. Système d'ancrage de suture (300) selon la revendication 1, dans lequel un organe de fixation (306) sur l'ancrage (302) passe à travers la boucle de suture affaissable (314) pour fixer la boucle affaissable (314) à l'ancrage (302).

3. Système d'ancrage de suture (300) selon la revendication 2, dans lequel l'organe de fixation est un montant (306) dans l'ancrage (302).

4. Système d'ancrage de suture (300) selon la revendication 3, dans lequel le nœud coulissant (312) est disposé en position proximale par rapport au montant (306).

5. Système d'ancrage de suture (300) selon la revendication 1, et comprenant en outre des saillies d'entrée en prise avec un os (308) autour de l'ancrage de suture (302).

6. Système d'ancrage de suture (300) selon la revendication 1, dans lequel l'ancrage de suture (302), la boucle affaissable (314) et la suture de tissu sont stériles et emballés dans une enceinte protégée contre les bactéries.

7. Système d'ancrage de suture (300) selon la revendication 1, dans lequel l'ancrage (302) a une forme cylindrique allongée lui permettant d'être reçu de manière fixe à l'intérieur d'un trou (326) percé dans un os (328).

8. Système d'ancrage de suture (300) selon la revendication 1, dans lequel le mécanisme de capture de suture (324) est une boucle de matériau flexible à travers laquelle la structure de tissu (316) peut être enfilée.
